# DEMANDE DE BREVET EUROPEEN

(11) **EP 4 670 626 A1**
(43) Date de publication de la demande: **31.12.2025**
(21) Numéro de dépôt: 25185609.2
(22) Date de dépôt: 26.06.2025
(51) Int. Cl.: A61B 5/03

(54) **DISPOSITIF ÉLECTRO-MÉDICAL ET KIT DE MESURE**

(30) Priorité: 27.06.2024 FR 2406979
(71) Demandeur: X-Pressure, 31400 Toulouse (FR)
(72) Inventeur: DE LAMY, Pierre-Edouard, 31000 TOULOUSE (FR)
(74) Mandataire: BARRE LAFORGUE

(57) **Abrégé**

L'invention concerne un dispositif électro-médical (10) configuré pour relayer, vers un terminal distant, un signal analogique représentatif des données physiologiques d'un patient telles que la pression intracrânienne ou à la pression artérielle du patient.

Le dispositif électro-médical (10) comporte ainsi :
- une batterie (14) rechargeable,
- un premier connecteur électronique (30) qui comprend un premier axe de connexion A-A, il permet de recevoir le signal analogique entrant,
- un connecteur d'alimentation (60) qui est relié électriquement à la batterie (14), le connecteur d'alimentation (60) comprenant un deuxième axe de connexion B-B,

La proximité spatiale entre les deux connecteurs (30, 60) et le croisement de leur axe de connexion A-A, B-B s'opposant au branchement simultané du premier connecteur électronique (30) et du connecteur d'alimentation (60).

## Description

### [Domaine technique]

L'invention concerne un dispositif électro-médical configuré pour relayer, vers un terminal distant, un signal analogique représentatif des données physiologiques d'un patient. Par exemple, les données physiologiques peuvent correspondre à la pression intracrânienne ou encore à la pression artérielle du patient etc. L'invention peut aussi concerner un kit de mesure de données physiologiques qui comprend un dispositif électro-médical conforme de l'invention et un capteur de données physiologiques.

### [État de la technique antérieure]

A l'heure actuelle, le développement d'un dispositif électro-médical, tel qu'un dispositif électroportatif pour relayer des données physiologiques d'un patient entre un capteur et un terminal distant (smartphone, ordinateur etc.), est soumis à différentes normes qui imposent des contraintes techniques. La plupart du temps ces contraintes techniques peuvent être prescrites pour des raisons de sécurité sanitaire liées à la biocompatibilité, la stérilisation, au vieillissement du dispositif, mais également à l'intégrité physique du patient vis-à-vis des risques électriques ou des risques électroacoustiques (Radio, CEM). La cybersécurité des données médicales est un autre volet important que doivent gérer les industriels pour les nouveaux dispositifs médicaux qui présentent des fonctionnalités de connectivités avec des réseaux locaux ou publics.

En ce qui concerne les dispositifs électroportatifs à usage médical, les normes établissent notamment que le fabricant doit faire en sorte que le courant alternatif à haute tension, provenant d'une prise électrique murale par exemple, ne puisse pas être transmis au patient car cela représenterait un danger de mort. La norme établit en particulier que le dispositif électro-médical doit permettre d'éviter tout court-circuit et tout passage d'électricité à haute tension depuis une source de courant à haute tension jusqu'au patient. La plupart du temps, la source de courant à haute tension est constituée par une prise électrique murale à laquelle le dispositif électro-médical est branché pour recharger sa batterie ou pour fonctionner. L'inversion de polarité, la limitation de la tension de sortie mais aussi la protection contre la surchauffe du dispositif électro-médical doivent être également traitées.

Afin de protéger les patients des risques électriques, certains fabricants ont équipé des dispositifs électroportatifs médicaux avec des composants électroniques de type convertisseur AC/DC, DC/DC, CC/CC, ou encore de différents systèmes d'isolation/protection du dispositif qui sont disposés sur la carte électronique. Cependant, ce type de composants électroniques est très onéreux et mais aussi très encombrant car ils doivent être dimensionnés de manière robuste pour protéger le patient. Il est ainsi difficile pour un fabricant de produire, à moindres coûts, des dispositifs compacts, et portatifs.

Pour résoudre ce problème en maitrisant les coûts de production, d'autres fabricants ont choisi d'équiper leurs dispositifs électroportatifs médicaux d'une batterie rechargeable amovible sans équiper les dispositifs de connecteur d'alimentation afin que la batterie ne puisse pas être rechargée sur secteur alors quelle est connectée au dispositif. Les fabricants doivent ainsi prévoir des caractéristiques spécifiques au niveau de la connectique pour éviter que la batterie ne puisse être rechargée alors qu'elle est connectée au dispositif. L'objectif est donc de contraindre les utilisateurs à sortir la batterie rechargeable du dispositif pour la recharger. La batterie doit ainsi être extraite du dispositif pour la recharger. Bien que cela réponde problème de sécurité, une batterie amovible présente des probabilités d'être égarée plus importante qu'une batterie interne non amovible. C'est d'autant plus le cas dans un environnement hospitalier où le matériel est utilisé par plusieurs praticiens voire par plusieurs équipes de praticiens. Par ailleurs, d'un point de vue de la conception, cela implique de ménager une ouverture et une porte dans le boitier pour extraire la batterie afin de la recharger et ensuite de la replacer dans le boitier le cas échéant. Cette porte devant dans certains cas être étanchéifiée pour éviter que des projections de fluide ne viennent endommager la batterie alors qu'elle se trouve dans son logement. Une telle solution s'avère donc insatisfaisante à bien des égards.

L'invention vise à pallier tout ou partie de ces inconvénients.

### [Exposé de l'invention]

L'invention vise à protéger le patient de tous risques électriques provenant d'un dispositif électro-médical tout en maitrisant les coûts de fabrication.

Pour cela l'invention concerne un dispositif électro-médical configuré pour relayer, vers un terminal distant, un signal électrique analogique représentatif des données physiologiques d'un patient telles que la pression intracrânienne ou la pression artérielle du patient, le signal électrique analogique étant produit par un capteur de physiologique électronique, le dispositif électro-médical comprenant un boitier qui comporte :
- une batterie rechargeable disposée dans un espace interne du boitier et fournissant de l'énergie électrique au dispositif électro-médical,
- un premier connecteur électronique qui comprend un premier axe de connexion A-A, le premier axe de connexion A-A permettant de connecter le premier connecteur électronique étant configuré, d'une part, pour être branché à un raccord relié au travers d'un câble au capteur physiologique électronique, et d'autre part, pour recevoir le signal analogique électrique entrant, et
- un connecteur d'alimentation qui est relié électriquement à la batterie et configuré pour être connecté à un raccord d'alimentation afin de transmettre un courant électrique à la batterie en vue de la recharger, le connecteur d'alimentation comprenant un deuxième axe de connexion B-B,
la premier connecteur électronique et le connecteur d'alimentation sont, d'une part, disposés spatialement de manière contiguë dans une zone de connexion du boitier, et d'autre part, orientés spatialement de sorte que le premier axe de connexion A-A soit sécant du deuxième axe de connexion B-B, le croisement du premier axe de connexion A-A et du deuxième axe de connexion B-B et la proximité spatiale des connecteurs s'opposant à un branchement simultané du premier connecteur électronique et du connecteur d'alimentation.

L'invention concerne donc un dispositif électroportatif à usage médical pour collecter et transmettre des données médicales, encore appelées, données physiologiques. Lorsque le dispositif électro-médical reçoit des données physiologiques, il est indirectement relié au patient au travers du capteur qui produit et transmet les données physiologiques. Par exemple, pour mesurer la pression intracrânienne d'un patient, le capteur utilisé est relié directement au patient au travers d'une ponction lombaire, le capteur étant connecté à l'aiguille de ponction lombaire. Le fait d'empêcher le branchement simultané des deux connecteurs permet d'éviter que le dispositif électro-médical ne soit pas relié simultanément au patient et à une source de courant alternatif haute tension. Les risques d'incidents électriques graves pouvant impliqués une atteinte du patient sont ainsi limités.

L'entrave mécanique est en particulier dû à l'agencement du connecteur d'alimentation par rapport au premier connecteur électronique. Plus particulièrement, leur proximité spatiale et le croisement de leur axe de connexion sont deux éléments qui participent à fournir une entrave mécanique lorsque l'un des deux connecteurs est branché à un raccord complémentaire extérieur qui est lui-même relié au travers d'un câble à un dispositif électro-médical. Le premier connecteur est donc configuré pour être branché avec un raccord complémentaire. Le raccord complémentaire peut être un connecteur mâle lorsque le premier connecteur est un connecteur femelle. Le problème de sécurité électrique est ainsi géré par une conception mécanique particulière de la zone de connexion du boitier. L'inventeur répond donc au problème technique sans utiliser de composants électroniques couteux et volumineux.

L'axe de connexion d'un connecteur correspond à l'axe selon lequel le raccord ou la fiche complémentaire du connecteur est branché sur le connecteur du dispositif électro-médical. La fiche ou le raccord est relié à un élément externe, tel qu'un capteur physiologique ou un transformateur, ce dernier étant lui-même connecté au réseau électrique via une prise secteur.

A noter que d'autres types de données telles que la pression intracrânienne, la pression artérielle, la pression vésicale, pression des loges musculaire du patient peuvent également être relayées par un dispositif électro-médical conforme d'un mode de réalisation de l'invention.

Dans des modes de réalisation, la distance entre le connecteur d'alimentation et le premier connecteur électronique peut être inférieure à 5 cm, de préférence, inférieure à 3 cm et encore de préférence inférieure à 2 cm ou encore inférieure à 1 cm. La proximité spatiale combinée avec une inclinaison d'au moins un des deux connecteurs fournissent un croisement des axes de connexion et créent ainsi l'entrave mécanique empêchant la connexion des deux connecteurs de manière simultanée.

Afin de permettre la connexion d'un maximum de capteurs de données physiologiques, le premier connecteur électronique peut être choisi parmi les connecteurs de données de types ODU MINI-MED^{®}, LEMO^{®}, Fisher^{®}, Smith^{®}, Attend^{®}, jack etc. En outre, le premier connecteur électronique peut être avantageusement un connecteur femelle. Cela permet de fournir un boitier dépourvu de protubérances qui est donc plus facilement transportable, et peut par exemple être emporté dans la poche d'une blouse. Pour les mêmes raisons avantageuses, le connecteur d'alimentation peut être un connecteur femelle. En outre, le connecteur d'alimentation peut être choisi parmi les connecteurs de types USB-C, USB-A, DIN etc. De préférence, le connecteur d'alimentation est de type USB-C.

En outre, la batterie rechargeable est préférentiellement non amovible du boitier. Par exemple, le boitier peut comprendre un logement spécifique contenant la batterie de manière permanente. Ce logement peut notamment être clos pour rendre la batterie non amovible. Ceci permet de répondre à la problématique de sécurité électrique tout en maitrisant les coûts de fabrication du boitier. En effet, le caractère non amovible ne contraint pas le fabricant à prévoir une ouverture dans le boitier, ce qui limite le nombre de pièces et simplifie la conception du boitier du dispositif électro-médical.

Dans des modes de réalisation, le premier connecteur électronique et le connecteur d'alimentation peuvent être ménagés dans une niche disposée en retrait ou décrochée par rapport à une paroi du boitier, la niche formant alors la zone de connexion du boitier. La niche étant en retrait de la paroi du boitier, elle participe à produire un encombrement mécanique de la zone de connexion en limitant notamment l'accès aux connecteurs.

Dans des modes de réalisation, le premier connecteur électronique est intégré sur un premier plan décroché par rapport à une paroi du boitier, le connecteur d'alimentation étant intégré sur un second plan décroché par rapport ladite paroi, le premier plan décroché et le second plan décroché étant sécants afin que les axes de connexion A-A et B-B se croisent. L'orientation spatiale particulière des plans décrochés participent à produire un encombrement spatial dans la zone de connexion qui va ensuite produire une entrave mécanique en coopérant, par exemple, avec la fiche mâle d'un raccord d'alimentation qui est déjà branchée sur le connecteur d'alimentation. La zone de connexion ne présente alors plus l'espace nécessaire pour brancher simultanément le premier connecteur électronique. De plus, les deux plans décrochés agencés dans les parois externes du boitier permettent de simplifier la conception du boitier et d'améliorer sa compacité notamment en réduisant le nombre de pièces du boitier. A l'inverse, dans une paroi plane, l'agencement incliné des capteurs implique l'utilisation d'adaptateurs au niveau de la paroi et/ou de structures de maintien intégrées dans le boitier pour maintenir les connecteurs en position désirée. Or, ces structures internes occupent de l'espace à l'intérieur du boitier qui n'est plus disponible pour la carte électronique du boitier. En pareil cas, il est donc nécessaire d'augmenter le volume du boitier pour contenir les structures internes de positionnement et la carte électronique. L'augmentation du volume du boitier n'est bien évidemment pas souhaitable, elle va à l'encontre d'un des objectifs de l'invention qui est de fournir une solution compacte et agile qui puisse être aisément utilisée en milieu hospitalier.

En particulier, le premier plan décroché et le second plan décrochés peuvent être disposés respectivement sur une première paroi et une deuxième paroi du boitier qui sont adjacentes et forment un angle β dont la valeur est inférieure à 160°. De préférence, la valeur de l'angle est comprise entre 60 et 150°, encore de préférence, la valeur de l'angle est comprise entre 80° et 140°. L'inclinaison d'une paroi par rapport à l'autre permet de limiter l'accès à la zone de connexion mais aussi de produire un croisement des axes de connexion des deux connecteurs. La première paroi et la deuxième paroi peuvent ainsi être agencées de manière à former une zone de connexion en L ou en demi U. Les axes de connexion A-A et B-B s'opposent respectivement par leur croisement et empêchent le branchement simultané d'un connecteur de données et du connecteur d'alimentation.

Dans des modes de réalisation, le second plan décroché peut créer une cassure avec le plan de ladite paroi du boitier, le premier plan décroché étant en retrait du plan de ladite paroi du boitier et s'étendant parallèlement à ladite paroi du boitier, le second plan décroché fait quant à lui la liaison entre ladite paroi du boitier et le premier plan décroché. Une telle configuration permet de donner à la zone de connexion une configuration de niche. Avantageusement, la paroi du boitier qui comporte la zone de connexion est une paroi latérale du boitier.

Dans des modes de réalisation, la zone de connexion peut être avantageusement disposée à proximité d'un angle du boitier. La position à proximité d'un angle du boitier permet d'utiliser les bords du boitier pour limiter l'accès à la zone de connexion.

Dans des modes de réalisation, la zone de connexion peut être disposée sur une paroi du boitier qui comporte une bordure saillante, ladite paroi comportant un méplat disposé en retrait de la bordure. La bordure saillante s'étend sur le pourtour de ladite paroi du boitier. Cette bordure contribue à limiter l'accès aux connecteurs. En particulier, le premier plan décroché et le second plan décroché forment avec la bordure saillante une niche en retrait du méplat. La bordure saillante accentue la profondeur de la niche et limite un peu plus l'accès aux connecteurs.

Dans des modes de réalisation, le premier plan décroché et le second décroché peuvent former un épaulement dans une paroi du boitier.

Dans des modes de réalisation, le premier connecteur électronique peut comporter une canule dont l'extrémité annulaire est disposée dans un plan surélevé par rapport au connecteur d'alimentation, le connecteur d'alimentation étant incliné par rapport à la canule. Une telle configuration permet de croiser les axes de connexion tout en réduisant la distance entre les deux connecteurs.

En particulier, le premier axe de connexion A-A et le deuxième axe de connexion B-B peuvent former un angle α dont la valeur est inférieure à 150°, de préférence, la valeur de l'angle α est inférieure à 145°, et encore de préférence, la valeur de l'angle α est inférieure à 140°. La valeur de l'angle α peut aussi être inférieure à 120° et de préférence, inférieure à 100°. La valeur de l'angle α peut également être supérieure à 20°, de préférence, la valeur de l'angle α est supérieure à 30°, encore de préférence la valeur de l'angle α est supérieure ou égale à 40°. La valeur de l'angle α peut ainsi être supérieure à 50°, et de préférence supérieure à 60°. De telles valeurs angulaires associées à une proximité spatiale des deux connecteurs permettent de créer une entrave mécanique au branchement d'un second connecteur alors que le premier connecteur est branché à un raccord extérieur au dispositif électro-médical.

Dans des modes de réalisation, le dispositif électro-médical peut comporter un deuxième connecteur électronique contiguë du connecteur d'alimentation, le deuxième connecteur électronique comprenant un troisième axe de connexion C-C, le troisième axe de connexion C-C étant sécant de l'axe de connexion B-B du connecteur d'alimentation, la proximité spatiale entre le connecteur d'alimentation et le troisième connecteur électronique et le croisement de leurs axes de connexion B-B, C-C s'opposant au branchement simultané du deuxième connecteur électronique et du connecteur d'alimentation. Ainsi, le dispositif électro-médical peut comporter deux connecteurs électroniques configurés pour transmettre des données, les deux connecteurs électroniques pouvant être connectés simultanément. Les deux connecteurs peuvent être de même type ou de type différent. Le deuxième connecteur électronique peut être choisi parmi les connecteurs de données de types ODU MINI-MED^{®}, LEMO^{®}, Fisher^{®}, Smith^{®}, Attend^{®}, jack etc. Le deuxième connecteur électronique est avantageusement de type différent du premier connecteur électronique. Par exemple, le deuxième connecteur électronique peut être de type jack alors que le premier et de type LEMO^{®}.

Dans tous les cas, ces deux connecteurs électroniques permettent de réaliser des analyses médicales complexes qui requièrent, par exemple, l'acquisition synchrone de deux types de données physiologiques tels qu'une électroencéphalographie et la pression intracrânienne du patient. Pour les mêmes raisons qui ont été précédemment exposées, le deuxième connecteur électronique peut être avantageusement un connecteur femelle.

Afin que les deux connecteurs électroniques puissent être connectés simultanément, le deuxième connecteur électronique peut être ménagé dans le même plan que le premier connecteur électronique. De plus, le deuxième connecteur électronique peut être disposé de manière adjacente par rapport au premier connecteur électronique.

Dans des modes de réalisation, le dispositif électro-médical peut comporter :
- des moyens électroniques comprenant un convertisseur numérique configuré pour convertir le signal électrique analogique entrant en signal numérique, et
- un émetteur/récepteur à distance configuré pour transmettre le signal numérique vers un terminal distant.

Dans des modes de réalisation, les moyens électroniques peuvent comporter une mémoire interne configurée pour stocker le signal numérique converti. Il est ainsi possible de stocker le signal entrant alors que le dispositif électro-médical n'est pas connecté à un terminal distant au moment de l'acquisition des données physiologiques.

Dans des modes de réalisation, les moyens électroniques peuvent comporter un accéléromètre configuré pour allumer le boitier et/ou appairer l'émetteur récepteur avec un terminal distant. Par exemple, deux tapotements sur une paroi de face du boitier peuvent déclencher la procédure d'appairage du dispositif de électro-médical à un terminal distant. Autre fonctionnalité envisageable, le fait d'allumer le dispositif électro-médical lorsque ce dernier est soumis à une agitation dépassant un certain seuil d'amplitude ou d'accélération.

Dans des modes de réalisation, le dispositif électro-médical peut comporter une interface homme/machine ménagée sur une paroi du boitier, l'interface homme/machine comprenant un ou plusieurs boutons, et/ou, un ou plusieurs indicateurs lumineux.

Dans des modes de réalisation, l'invention peut se rapporter à un kit de mesure de données physiologiques d'un patient telles que la pression intracrânienne ou la pression artérielle d'un patient, le kit de mesure comprenant un dispositif électro-médical conforme d'un mode de réalisation de l'invention et un capteur physiologique configuré, d'une part, pour mesurer la pression d'un liquide physiologique d'un patient, et d'autre part, pour produire un signal électrique analogique représentatif de ladite pression. En particulier, le dispositif électro-médical est avantageusement associé à un capteur de pression intracrânienne qui est relié au liquide céphalorachidien, ce dernier étant rendu accessible au travers d'une procédure de ponction lombaire.

### [Description des dessins]

D'autres caractéristiques et avantages de l'invention apparaîtront encore à la lecture de la description qui va suivre. Celle-ci est purement illustrative et doit être lue en regard des dessins annexés sur lesquels :
[Fig. 1] est une représentation en perspective d'un dispositif électro-médical conforme d'un mode de réalisation de l'invention.
[Fig. 2] est une représentation du boitier du dispositif de la figure 1, les connecteurs n'étant pas assemblés.
[Fig. 3] est une représentation d'un dispositif électro-médical conforme d'un mode de réalisation de l'invention, un premier connecteur électronique du boitier étant branché au connecteur mâle d'un câble électronique, la configuration du boitier et le connecteur mâle s'opposant au branchement du connecteur d'alimentation.
[Fig. 4] est une représentation d'un dispositif électro-médical conforme d'un mode de réalisation de l'invention, le connecteur d'alimentation est branché à un connecteur mâle d'un câble alimentation, la configuration du boitier et le connecteur mâle s'opposant qui s'oppose au branchement simultané du connecteur électronique.
[Fig. 5] est une représentation de la zone de connexion d'un dispositif électro-médical conforme d'un mode de réalisation de l'invention.
[Fig. 6] est une représentation de la zone de connexion d'un dispositif électro-médical conforme d'un autre mode de réalisation de l'invention.
[Fig. 7] est une représentation du dispositif électro-médical de la figure 6, un premier connecteur électronique du boitier étant branché au connecteur mâle d'un câble électronique, la configuration du boitier et le connecteur mâle s'opposant au branchement du connecteur d'alimentation.
[Fig. 8] est une représentation d'un système de transmission des données physiologiques d'un patient utilisant un dispositif électro-médical conforme de l'invention.
[Fig. 9] est une représentation schématique d'une coupe transversale de la zone de connexion d'un dispositif électro-médical conforme d'un mode de réalisation de l'invention.

### [Description des modes de réalisation]

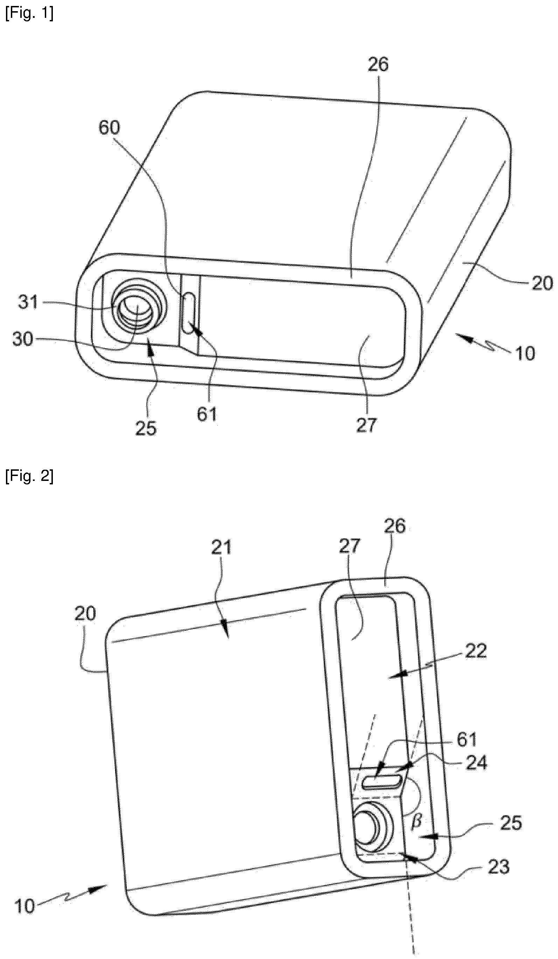

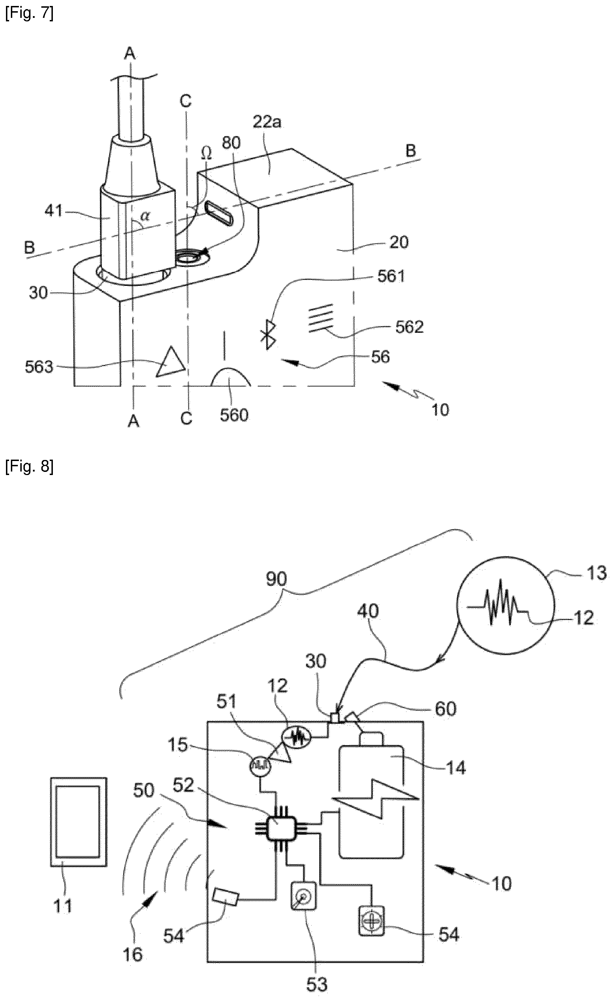

En référence aux figures 1 à 9, l'invention concerne un dispositif électro-médical 10 médical. Le dispositif électro-médical 10 est en particulier configuré pour relayer des données physiologiques d'un patient vers un terminal distant 11 tel que cela est illustré à la figure 8. Le terminal distant 11 peut être un smartphone, une tablette numérique, un ordinateur ou tout autre dispositif de traitement de données qui comporte des fonctionnalités de connectivité, de préférence sans fil, et une interface homme machine permettant d'accéder aux données relayées par le dispositif électro-médical 10.

Les données physiologiques peuvent être en particulier relayées sous forme d'un signal électrique 12 produit par un capteur 13 de données physiologiques. La plupart du temps les capteurs 13 utilisés produisent un signal électrique analogique correspondant aux données physiologiques d'un patient qu'ils mesurent. Par exemple, les inventeurs ont décrit un capteur permettant de mesurer la pression intracrânienne d'un patient au travers de la pression du liquide céphalorachidien (LCR) comme cela est décrit dans le document WO2023/186817. Le capteur est ainsi connecté à une aiguille de ponction lombaire pour être mis au contact du LCR. La pression du LCR est mesurée au travers d'un volume d'air contrôlé qui est intercalé entre une cellule piézoélectrique du capteur et le LCR. La cellule piézoélectrique retranscrit alors la pression du LCR en produisant un signal électrique analogique. A noter que d'autres types de données telles que la pression artérielle, la pression vésicale, la pression des loges musculaire du patient peuvent également être relayées par le dispositif électro-médical 10 conformément à des modes de réalisation de l'invention. Le capteur physiologique 13 est donc plus particulièrement un capteur de type électronique. Le signal électrique analogique que produit ce type de capteur 13 est un signal d'une durée déterminé qui peut comprendre plusieurs composantes. Par exemple, dans le cas de la mesure de la pression intracrânienne le signal électrique analogique produit par le capteur comprend : la dynamique intracrânienne, le signal cardiaque, le signal respiratoire et probablement des ondes B qui sont associées aux cycles vasomoteurs.

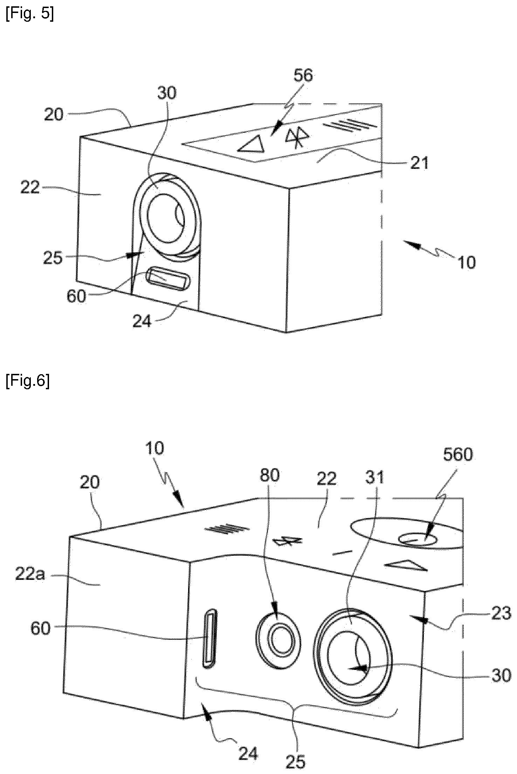

Comme cela est illustré aux figures 1 à 7, le dispositif électro-médical 10 comporte un boitier 20 qui comprend une forme géométrique sensiblement quadrangulaire. Le boitier 20 comporte ainsi deux parois de face 21 qui s'opposent l'une à l'autre et des parois latérales 22 qui ferment le boitier et s'étendent à la périphérie des parois de face 21. Le terme sensiblement signifie que les arrêtes et les angles du quadrangulaire peuvent être arrondies comme cela est visible aux figures 5 à 7. De plus, dans le mode de réalisation des figures 5 et 6, une paroi latérale 22a présente un décroché. Néanmoins, le boitier peut prendre toutes sortes de forme dès lors qu'il comprend une zone de connexion conforme d'un mode de réalisation de l'invention.

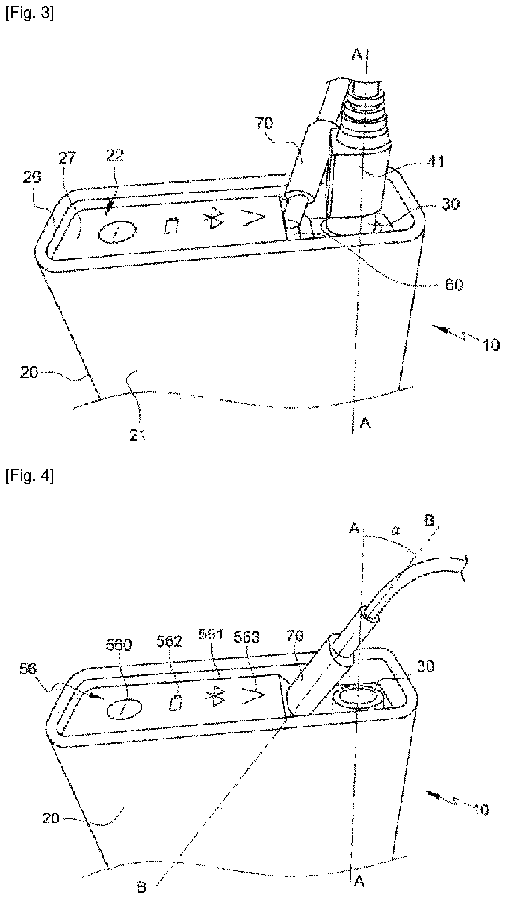

D'ailleurs, le boitier 20 comprend, dans cette zone de connexion, un premier connecteur électronique 30. Ici, le premier connecteur 30 est un connecteur femelle, il permet de brancher un câble de données 40 à l'aide d'un connecteur mâle 41. Ceci est en particulier illustré aux figures 3, 7 et 8. Le flux entrant de données vers le dispositif électro-médical 10 est en particulier représenté à la figure 8 par des flèches qui sont représentées sur le câble 40.

Selon un mode de réalisation illustré notamment aux figures 4 et 7, le connecteur premier connecteur 30 comprend un premier axe de connexion A-A. L'axe de connexion A-A permet de connecter le premier connecteur électronique 30 à un connecteur mâle 41 qui est relié via un câble 40 au capteur 13 physiologique, ce dernier produisant le signal analogique entrant. Dans cet exemple, le premier connecteur 30 est de type connecteur de données, en particulier, il s'agit d'un connecteur de données médicales tel que les connecteurs ODU MINI-MED^{®}, LEMO^{®}, Fisher^{®}, Smith^{®}, Attend^{®}. Néanmoins, le connecteur de données peut être choisi dans un type de connecteurs généralistes tel que la prise jack. La prise jack peut permettre de brancher au dispositif électro-médical 10 un appareil de type électroencéphalographe (EEG) ou un appareil de type MEG qui permet de réaliser une magnétoencéphalographie.

Selon un mode de réalisation, le dispositif électro-médical 10 peut comprendre une batterie rechargeable 14 disposée dans un espace interne du boitier 20, par exemple un logement spécifique comportant des connectiques lui permettant de distribuer l'énergie électriques aux moyens électroniques 50 que comporte le dispositif électro-médical 10. La batterie 14 est de préférence inamovible. A cet effet, le logement peut être clos.

Selon le mode de réalisation illustré notamment à la figure 8, les moyens électroniques 50 peuvent comporter un convertisseur numérique 51 configuré pour convertir le signal analogique 12 entrant en signal numérique 15. Avantageusement, les moyens électroniques 50 peuvent également comprendre un amplificateur. L'amplificateur améliore, avantageusement, le rapport signal/bruit du signal analogique 12 avant sa conversion. Le dispositif électro-médical 10 peut ainsi être considéré comme un modulateur et un boitier relai de données physiologiques. Par ailleurs, les moyens électroniques 50 peuvent comporter un microprocesseur 52 couplé à une mémoire 53 pour stocker, au moins temporairement, le signal numérique 15. Les moyens électroniques 50 peuvent comprendre plusieurs mémoires de type Eprom, Fram, SD.

Comme illustré à la figure 8, les moyens électroniques 50 peuvent également comprendre un émetteur/récepteur 54 sans fil configuré pour transmettre le signal numérique 15 vers le terminal distant 11. L'émetteur/récepteur 54 peut être configuré pour émettre un signal de champ proche tel que le NFC, le Bluetooth et le RFID ou encore un signal de type WIFI, Radio etc. Avantageusement, l'émetteur/récepteur 54 est choisi de type Bluetooth afin de permettre une connectivité à une distance de quelques mètres avec les appareils connectés les plus courants tels que les smartphones, tablettes voire les ordinateurs portables.

Le microprocesseur 52 commande ainsi le transfert du signal numérique 15 qui a été converti vers un terminal distant 11 ou vers la mémoire 53 comme cela est illustré à la figure 8. Par exemple, lorsque le dispositif électro-médical 10 est relié à un capteur 13 de données physiologiques, et que le dispositif électro-médical 10 n'est pas connecté à un terminal distant 11, le microprocesseur 52 va stocker le signal numérique 15 dans la mémoire 53. Le signal numérique 15 peut être ainsi stocké jusqu'à la prochaine connexion à un terminal distant 11. Comme l'acquisition des données physiologiques requiert des actes médicaux plus ou moins invasifs, par exemple une ponction lombaire, il est important que les données physiologiques mesurées par le capteur 13 puissent être conservées dans le dispositif électro-médical 10 en toutes circonstances et même lorsque le dispositif électro-médical 10 n'est pas en mesure de transmettre les données vers un terminal distant 11.

Le microprocesseur 52 peut également être configuré pour crypter le signal sans fil 16 qui ensuite transmis vers le terminal distant 11. Ceci permet de respecter les normes de confidentialité des données médicales personnelles.

En outre, les moyens électroniques 50 peuvent comprendre un accéléromètre configuré pour allumer le boitier et/ou appairer l'émetteur/récepteur avec un terminal distant. Par exemple, l'accéléromètre peut être configuré pour éveiller les moyens électroniques 50 du dispositif électro-médical 10 dès lors qu'une certaine agitation est produite. Selon une autre possibilité qui peut être associée ou non avec la première fonctionnalité de l'accéléromètre 54, ce dernier peut être configuré pour appairer l'émetteur/récepteur 54 avec un terminal distant à la suite d'un stimulus spécifique. Le stimulus peut par exemple correspondre à deux tapotements sur une face du boitier 20.

Comme cela est visible aux figures 4 à 7, le dispositif électro-médical 10 peut comprendre une interface homme/machine 56 qui est ici ménagée sur une paroi du boitier 20. Dans cet exemple, l'interface homme/machine 56 comprend un bouton on/off 560 et des indicateurs lumineux 561, 562, 563. Un premier indicateur 561 lumineux peut informer l'utilisateur si le dispositif électro-médical 10 est appairé à un terminal distant ou en procédure d'appairage. Ici, le premier indicateur 561 porte le sigle de la connectivité Bluetooh. Un deuxième indicateur 562 lumineux peut informer de l'état de charge ou du chargement en cours de la batterie, une batterie étant représentée à cet effet. Un troisième indicateur 563 peut désigner le connecteur 30 à utiliser pour brancher le raccord du capteur 13. Il est à noter que le bouton 560 on/off peut également intégrer un indicateur lumineux qui informe l'utilisateur de l'état de fonctionnement marche (on) ou arrêt (off) du dispositif électro-médical 10.

La batterie 14 n'est pas visible sur les figures 1 à 7, elle est toutefois représentée symboliquement à la figure 8. En revanche, le boitier 20 comporte, dans la zone de connexion, un connecteur d'alimentation 60 qui est relié électriquement à la batterie 14. Le connecteur 60 peut être également relié aux composants électroniques gérant le rechargement de la batterie 14. Le connecteur d'alimentation 60 est visible sur les figures 1 à 9. Ici, le connecteur d'alimentation 60 est un connecteur femelle qui est configuré pour être connecté à un raccord d'alimentation 70 mâle du même type. Le connecteur d'alimentation 60 permet de transmettre un courant électrique à la batterie 14 en vue de la recharger. Le connecteur d'alimentation 60 peut être choisi parmi les connecteurs de types USB-C, USB-A, DIN etc. Dans l'exemple des figures 1 à 7, le connecteur USB-C est avantageusement choisi car il allie puissance de transmission, facilité d'utilisation par son caractère réversible, et est également car il est très répandu à l'heure de la rédaction de ce document. Comme cela est illustré aux figures 2 et 7, le connecteur d'alimentation 60 comprend un deuxième axe de connexion B-B qui s'étend longitudinalement selon l'axe de connexion du raccord d'alimentation 70 mâle.

Comme illustré aux figures 1 à 8, le premier connecteur électronique 30 et leconnecteur d'alimentation 60 peuvent être disposés à proximité l'un de l'autre. En pratique, la distance entre ces deux connecteurs 30, 60 peut être inférieure à 5 cm, de préférence, inférieure à 3 cm et encore de préférence inférieure à 2 cm ou encore inférieure à 1 cm. Les deux connecteurs 30, 60 sont donc disposés spatialement de manière contiguë dans la zone de connexion du boitier 20. De plus, les deux connecteurs 30, 60 peuvent être orientés spatialement de sorte que leur axe connexion A-A, B-B soient sécants l'un à l'autre. En particulier, les deux axes de connexion A-A, B-B forment un angle α de valeur déterminée tel que cela est illustré aux figures 4 et 7. Dans l'exemple de la figure 4, l'angle α a valeur d'environ 40°, alors que l'exemple de la figure 7 montre un angle α dont la valeur est de l'ordre de 90°. Le croisement des axes de connexion A-A, B-B et la proximité spatiale des connecteurs 30, 60 coopèrent pour s'opposer à un branchement simultané de ces deux connecteurs 30, 60 tel que cela est illustré à la figure 3. Dans cette figure, le raccord mâle 41 du câble de données 40 est branché sur le premier connecteur électronique 30 selon l'axe A-A qui s'étend de manière perpendiculaire par rapport au plan de la paroi 22 du boitier 20. On voit ainsi sur cette figure 3, que le raccord mâle 41 crée avec la configuration de la zone de connexion une entrave mécanique au branchement du raccord d'alimentation 70 sur le connecteur d'alimentation 60. La proximité spatiale entre les connecteurs 30, 60 et le croisement des axes A-A, B-B fournissent une zone de connexion réduite et encombrée. Ainsi, lorsqu'un raccord mâle est branché à l'un des connecteurs 30, 60, il entrave le branchement simultané de l'autre connecteur 30, 60 par la configuration de la zone de connexion. La figure 4 montre que lorsque le raccord d'alimentation 70 est branché au connecteur d'alimentation 60, il produit le même effet d'entrave mécanique. Selon ce mode de réalisation, le raccord d'alimentation 70 croise l'axe A-A selon l'angle α et empêche clairement le branchement simultané du raccord compatible avec le connecteur électronique 30.

Selon la configuration de la zone de connexion, il est possible d'obtenir un tel effet empêchant deux connexions simultanées lorsque la valeur de l'angle α est inférieure à 150°, de préférence, la valeur de l'angle α est inférieure à 145°, et encore de préférence, la valeur de l'angle α est inférieure à 140°. La valeur de l'angle α peut également être supérieure à 20°, de préférence, la valeur de l'angle α est supérieure à 30°, encore de préférence la valeur de l'angle α est supérieure ou égale à 40°.

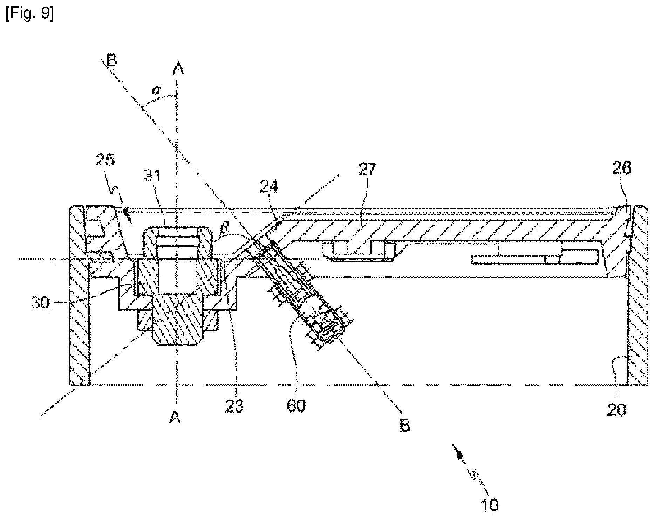

Selon un mode de réalisation illustré aux figures 1 à 7, le premier connecteur électronique 30 est intégré sur un premier plan 23 qui est décroché d'une paroi 22, 22a du boitier 20 alors que le connecteur d'alimentation 60 est intégré sur un second plan décroché 24 de ladite paroi 22, 22a. Les deux plans 23, 24 sont sécants afin que les axes de connexion A-A, B-B se croisent. Les deux plans 23, 24 sont intégrés aux parois 21, 22, 22a du boitier 20 et forment la zone de connexion. Les plans 23, 24 peuvent en quelques sortes être considérés comme des parois du boitier 20, notamment dans le cadre des figures 6 et 7. Dans l'exemple de la figure 9, on voit que l'embase du connecteur 30 comprend une paroi qui s'étend dans le plan 23.

Selon un mode de réalisation illustré à la figure 2, le boitier 20 comporte des plans décrochés 23, 24 et les ouvertures prévues respectives pour l'assemblage des connecteurs 30, 60. Sur cette figure les plans décrochés 23, 24 forment un angle β dont la valeur est supérieure à 90°. En particulier, la valeur de l'angle β est ici comprise entre 160 et 140°. Dans ce mode de réalisation, l'inclinaison des plans décrochés 23, 24 l'un par rapport à l'autre fournit des axes de connexion sécants tels que décrits précédemment.

Comme illustré aux figures 1 à 7 et 9, les connecteurs 30, 60 sont disposés dans une niche 25 disposée en retrait ou décrochée par rapport à une paroi 22, 22a du boitier 20. La niche 25 forme alors la zone de connexion du boitier 20. En particulier, le second plan décroché 24 crée une cassure avec le plan d'une paroi 22, 22a du boitier 20. Le premier plan décroché 23 est en retrait du plan de ladite paroi 22, 22a et s'étend parallèlement à ladite paroi 22, 22a du boitier 20. Le second plan 24 fait donc la liaison entre ladite paroi 22, 22a du boitier 20 et le premier plan 23.

Un premier mode de réalisation est illustré aux figures 1 à 4 et 9, la paroi 22 au niveau de de laquelle est agencée la zone de connexion comporte une bordure saillante 26 qui s'étend sur le pourtour de la paroi 22. La paroi 22 comporte un méplat 27 qui disposé en retrait de la bordure 26. Ici, la bordure 26 coopère avec les plans décrochés 23, 24 pour former une niche 25 plus difficile d'accès, ce qui augmente les contraintes s'opposant au branchement simultané des deux connecteurs 30, 60. Le méplat 27 participe également à l'entrave mécanique, puisque le second plan décroché 24 crée une cassure dans le méplat 27 à proximité d'une extrémité de la paroi 22. L'extrémité de la paroi 22 est située à proximité d'un angle du boitier 20, l'encombrement de la zone de connexion est donc augmenté par la présence la bordure 26 qui entoure le méplat 27 sur trois côtés à cet emplacement.

Le méplat 27 peut servir pour l'intégration de l'interface homme machine tel que cela est illustré aux figures 3 et 4. Le méplat 27 peut également être utilisé par le fabricant pour apporter des informations techniques et législatives sur le dispositif électro-médical 10.

La figure 9 montre une vue en coupe du premier mode de réalisation. Il est possible de voir que les orientations respectives des axes de connexion A-A, B-B sont conférées par l'intégration de chaque connecteur 30, 60 au niveau de la niche 25 du boitier 20 mais aussi de la carte électronique (non visible). C'est donc la configuration de la zone de connexion du boitier 20 qui permet le croisement desdits axes. En particulier, l'inclinaison du second plan décroché 24 fournit un plan incliné dans lequel est ménagée l'ouverture 61 du connecteur d'alimentation 60. Comme cela est visible, le premier plan décroché 23 est parallèle du méplat 27 et est disposé en contrebas de ce dernier dans la niche 25. Le second plan décroché 24 forme ainsi le même angle β avec le méplat 27 et le premier plan décroché 23. L'angle β caractérise en quelques sortes l'inclinaison du connecteur d'alimentation 60 par rapport connecteur électronique 30 afin que le boitier 20 dispose d'axes de connexion A-A, B-B croisés qui s'opposent au branchement simultané de ces deux connecteurs 30, 60. Notons que les orientations des connecteurs 30, 60 sont conservées dans leur intégration sur la carte électronique. Comme illustré à la figure 9, chaque connecteur 30, 60 s'étend selon son axe de connexion A-A, B-B respectif à l'intérieur du boitier 20, leur orientation est donc conservée dans leur intégration sur la carte électronique.

Selon un deuxième mode de réalisation illustré à la figure 5, la zone de connexion peut se présenter sous la forme d'une niche 25 qui s'étend dans la paroi latérale 22 en forme de U. La niche 25 est disposée sur l'axe médian transversal de la paroi 22. Ici, le second plan décroché 24 crée une cassure depuis un bord de la paroi 22 et est incliné en direction du premier plan décroché 23. Ce dernier n'est pas visible car le connecteur 30 est saillant du premier décroché plan 23 et occupe une grande partie de l'espace disponible.

En particulier, le connecteur 30 comporte une canule dont l'extrémité annulaire 31 affleure le plan de la paroi 22. Ici, le connecteur d'alimentation 60 comporte une ouverture 61 qui correspond au port de connexion, l'ouverture 61 étant disposée sous le plan dans lequel s'étend l'extrémité annulaire 31 du connecteur 30. De plus, l'ouverture 61 s'étend dans le second plan décroché 24 qui est incliné par rapport à l'extrémité annulaire 31 qui s'étend dans un plan parallèle au premier plan décroché 23. Cette inclinaison de l'ouverture 61 fournit des axes de connexion A-A, B-B croisés qui ne permettent pas le branchement simultané des deux connecteurs 30, 60 par la proximité spatiale des connecteurs 30, 60. Une telle configuration est également présente dans le mode de réalisation illustré aux figures 1 à 4. Selon ce mode réalisation l'extrémité annulaire 31 se trouve sensiblement dans le plan du méplat 27 de la paroi 22. L'ouverture 61 est disposée sous le plan du méplat 27 comme cela est particulièrement visible à la figure 2. En particulier, l'ouverture 61 est ménagée dans le second plan décroché 24 et dispose donc d'une inclinaison par rapport au connecteur 30 et sa canule.

A noter que sur la figure 5, l'interface homme/machine 56 est ménagée sur la paroi de face 21 du boitier 20.

Selon un troisième mode de réalisation illustré aux figures 6 et 7, le second plan décroché 24 est agencé de manière perpendiculaire par rapport, d'un côté, à la paroi 22a, et d'un autre côté, au premier plan décroché 23. On peut ainsi dire que la paroi 22a présente un décroché qui forme la niche 25 de la zone de connexion. La niche 25 de la zone de connexion présente ainsi une forme en L ou en demi U. Le connecteur 60 étant disposé dans le second plan déroché 24 alors que le connecteur 30 est disposé dans le premier plan décroché 23. Cet agencement contribue à positionner les deux axes de connexion A-A, B-B de manière sensiblement perpendiculaire à quelques degrés prés. Ici, l'extrémité annulaire 31 du connecteur 30 affleure la face du premier plan décroché 23 et l'ouverture 61 du connecteur d'alimentation 60 se trouve dans un plan supérieur au premier plan décroché 23.

Bien que cette organisation spatiale des connecteurs 30, 60 diffère de celles des autres modes de réalisation, la corrélation entre la proximité spatiale des deux connecteurs 30, 60 et le croisement des axes de connexion A-A, B-B des connecteurs 30, 60 s'opposent au branchement simultané des deux connecteurs 30, 60. Comme cela est décrit précédemment, la distance entre les deux connecteurs 30, 60 peut être inférieure à 5 cm, de préférence, inférieure à 3 cm et encore de préférence inférieure à 2 cm, mieux la distance peut être inférieure à 1 cm.

Comme illustré aux figures 6 et 7, le boitier 20 peut comporter un deuxième connecteur électronique 80. Ici, le connecteur 80 est de type prise jack. Toutefois, on peut utiliser en remplacement tous types de connecteur de données, en particulier, ceux utilisés dans le domaine de la santé et du médical tels que ODU MINI-MED^{®}, LEMO^{®}, Fisher^{®}, Smith^{®}, Attend^{®} etc. Comme illustré aux figures 6 et 7, le connecteur 80 est également contiguë du connecteur d'alimentation 60. La proximité spatiale entre le deuxième connecteur électronique 80 et le connecteur d'alimentation 60 est du même ordre que celle entre le premier connecteur électronique 30 et le connecteur d'alimentation 60.

Dans cet exemple, le connecteur 80 est adjacent du connecteur 30, ils sont tous deux disposés sur le premier plan décroché 23. Plus précisément, le deuxième connecteur électronique 80 est intercalé entre le connecteur 30 et le second plan décroché 24 sur lequel est ménagé le connecteur d'alimentation 60. Cette configuration confère au connecteur 80 un troisième axe de connexion C-C qui est parallèle de l'axe de connexion A-A du premier connecteur électronique 30. Par conséquent, le troisième axe de connexion C-C est perpendiculaire de l'axe de connexion B-B du connecteur d'alimentation 60. L'angle Ω entre les axes de connexion B-B, C-C est donc identique à l'angle α qui est formé par les axes de connexion A-A, B-B. A nouveau, la proximité spatiale entre les connecteurs 60, 80 et le croisement de leurs axes de connexion B-B, C-C s'opposent à l'utilisation simultanée de ces deux connecteurs 60, 80.

En revanche, la position adjacente des connecteurs électroniques 30, 80 sur un même plan, fournit des axes de connexion A-A, C-C parallèles entre eux. De plus, l'écartement entre les deux connecteurs 30, 80 est, certes faible, par exemple inférieur à 5 cm ou plus faible encore inférieur à 3 cm et même inférieure à 1 cm, toutefois, leur position dans un même plan autorise un branchement simultané de ces deux connecteurs électroniques 30, 80. Le fait de disposer de deux connecteurs électroniques identiques ou différents permet de relier le dispositif électro-médical 10 à deux capteurs physiologiques distincts de manière à collecter de types de données différentes de manière synchrone.

Ceci peut être utile lorsqu'un praticien a besoin de réaliser des examens complexes qui nécessitent l'acquisition simultanée de type de données tel que la pression intracrânienne et l'impédance cérébrale d'un patient. L'impédance cérébrale correspond à l'opposition que produisent les tissus du cerveau au passage d'un courant électrique. L'impédance cérébrale peut être mesurée en utilisant plusieurs techniques comme l'électroencéphalographie (EEG) ou la magnétoencéphalographie (MEG). Des capteurs placés sur le crâne du patient peuvent être utilisés pour mesurer les signaux électriques ou électromagnétiques du cerveau.

Les mesures simultanées de l'impédance cérébrale et de la pression intracrânienne permettent aux cliniciens d'obtenir une évaluation plus complète de l'état neurologique du patient. Cela peut être utile pour surveiller ou diagnostiquer plusieurs pathologies ou anomalies liées au cerveau : hydrocéphalie, traumatisme crânien, tumeurs cérébrales, œdèmes cérébraux, infections cérébrales, hémorragies intracrânienne, crises hypertensives intracrâniennes, syndrome de tension intracrânienne idiopathique etc... Les capteurs EEG ou EMG peuvent comprendre des raccords différents, par exemple, le capteur EEG comprend généralement un raccord mâle de type jack alors que le capteur de pression intracrânienne peut être par exemple de type ODU MINI-MED^{®}. Ainsi, les deux connecteurs 30, 80 illustrés aux figures 6 et 7 permettent de recueillir les deux acquisitions simultanées au sein du dispositif électro-médical 10. Les deux signaux peuvent être traités simultanément ou l'un après l'autre par les moyens électroniques puis transmis vers le terminal distant 11 comme illustré à la figure 8.

Il est à noter que les deux précédents modes de réalisation décrits aux figures 1 à 6 et 9 peuvent également comprendre deux voire trois connecteurs électroniques. Ceci pour collecter des données qui sont issues de plusieurs capteurs physiologiques réalisant des acquisitions synchrones. Pour parvenir à introduire plus de connecteurs électroniques en conservant les avantages de l'invention, la zone de connexion peut être agrandie et notamment le premier plan décroché 23 sur lequel est disposé le connecteur 30. Le connecteur supplémentaire est ménagé de manière adjacente au connecteur 30, à proximité de ce dernier pour que le connecteur d'alimentation 60 ne puisse pas être branché simultanément.

Comme illustré à la figure 8, un selon un mode de réalisation l'invention peut aussi concerner un kit de mesure 90 de données physiologiques d'un patient. Ce kit de mesure 90 peut être adapté à pour mesurer la pression intracrânienne ou la pression artérielle d'un patient. Pour cela, le kit comprend un dispositif électro-médical 10 conforme d'un mode de réalisation de l'invention et un capteur physiologique 13. De préférence, le capteur physiologique 13 est configuré pour mesurer la pression d'un liquide physiologique d'un patient tel que cela a été précédemment expliqué. Néanmoins, le kit peut comprendre le dispositif électro-médical 10 conforme d'un mode de réalisation de l'invention associé à un autre capteur de données physiologiques tels qu'un capteur de pression.

## Revendications

1. Dispositif électro-médical (10) configuré pour relayer, vers un terminal distant, un signal électrique analogique représentatif des données physiologiques d'un patient telles que la pression intracrânienne ou à la pression artérielle du patient, le signal électrique analogique étant produit par un capteur de physiologique électronique, le dispositif électro-médical (10) comprenant un boitier (20) qui comporte :
- une batterie (14) rechargeable disposée dans un espace interne du boitier (20) et fournissant de l'énergie électrique dispositif électro-médical (10),
- un premier connecteur électronique (30) qui comprend un premier axe de connexion A-A, le premier axe de connexion A-A permettant de connecter le premier connecteur électronique (30) étant configuré, d'une part, pour être branché à un raccord relié au travers d'un câble (40) au capteur physiologique électronique, et d'autre part, pour recevoir le signal analogique électrique entrant,
- un connecteur d'alimentation (60) qui est relié électriquement à la batterie (14) et est configuré pour être connecté à un raccord d'alimentation afin de transmettre un courant électrique à la batterie (14) en vue de la recharger, le connecteur d'alimentation (60) comprenant un deuxième axe de connexion B-B,
la premier connecteur électronique (30) et le connecteur d'alimentation (60) sont, d'une part, disposés spatialement de manière contiguë dans une zone de connexion du boitier (20), et d'autre part, orientés spatialement de sorte que le premier axe de connexion A-A soit sécant du deuxième axe de connexion B-B, le croisement du premier axe de connexion A-A et du deuxième axe de connexion B-B et la proximité spatiale des connecteurs (30, 60) s'opposant au branchement simultanée du premier connecteur électronique (30) et du connecteur d'alimentation (60).

2. Dispositif électro-médical (10) selon la revendication 1, dans lequel, le premier connecteur électronique (30) et le connecteur d'alimentation (60) sont ménagés dans une niche (25) disposée en retrait ou décrochée par rapport à une paroi (22, 22a) du boitier (20), la niche (25) formant alors la zone de connexion du boitier (20).

3. Dispositif électro-médical (10) selon l'une des revendications 1 et 2, dans lequel, le premier connecteur électronique (30) est intégré sur un premier plan décroché (23) par rapport à une paroi (22, 22a) du boitier (20), le connecteur d'alimentation (60) étant intégré sur un second plan décroché (24) par rapport ladite paroi (22, 22a), le premier plan décroché (23) et le second plan décrochés (24) étant sécants afin que les axes de connexion A-A et B-B se croisent.

4. Dispositif électro-médical (10) selon la revendication 3, dans lequel, le second plan décroché (24) crée une cassure avec le plan de ladite paroi (22, 22a) du boitier (20), le premier plan décroché (23) étant en retrait du plan de ladite paroi et s'étendant parallèlement à ladite paroi, le second plan décroché (24) faisant la liaison entre ladite paroi et le premier plan décroché (23).

5. Dispositif électro-médical (10) selon l'une des revendications 1 à 4, dans lequel, la zone de connexion est disposée sur une paroi (22, 22a) du boitier (20) qui comporte une bordure saillante (26), ladite paroi comportant un méplat (27) disposé en retrait de la bordure saillante (26).

6. Dispositif électro-médical (10) selon les revendications 4 et 5, dans lequel, le premier plan décroché (23) et le second plan décroché (24) formant avec la bordure saillante (26) une niche (25) en retrait du méplat (27).

7. Dispositif électro-médical (10) selon l'une des revendication 3 et 4, dans lequel, le premier plan décroché (23) et le second plan décroché (24) forment un épaulement dans une paroi (22, 22a) du boitier (20).

8. Dispositif électro-médical (10) selon l'une de revendications 1 à 7, dans lequel, le premier connecteur électronique (30) comporte une canule dont l'extrémité annulaire (31) est disposée dans un plan surélevé par rapport au connecteur d'alimentation (60), le connecteur d'alimentation (60) étant incliné par rapport à la canule.

9. Dispositif électro-médical (10) selon l'une des revendications 1 à 8, dans lequel, le premier axe de connexion A-A et le deuxième axe de connexion B-B forment un angle α dont la valeur est inférieure à 150°, de préférence, la valeur de l'angle α est inférieure à 145°, et encore de préférence, la valeur de l'angle α est inférieure à 140°.

10. Dispositif électro-médical (10) selon l'une des revendications 1 à 9, dans lequel, le boitier (20) comporte un deuxième connecteur électronique (80) contiguë du connecteur d'alimentation (60), le deuxième connecteur électronique (80) comprenant un troisième axe de connexion C-C, le troisième axe de connexion C-C étant sécant de l'axe de connexion B-B du connecteur d'alimentation (60), la proximité spatiale entre le connecteur d'alimentation (60) et le deuxième connecteur électronique (80) et le croisement de leurs axes de connexion B-B, C-C s'opposant au branchement simultané du deuxième connecteur électronique (80) et du connecteur d'alimentation (60).

11. Dispositif électro-médical (10) selon la revendication 10, dans lequel, le deuxième connecteur électronique (80) est disposé dans le même plan que le premier connecteur électronique (30).

12. Dispositif électro-médical (10) selon l'une des revendications 1 à 11, qui comporte des moyens électroniques (50) comprenant :
- un convertisseur numérique (51) configuré pour convertir le signal électrique analogique entrant en signal numérique, et
- un émetteur/récepteur (54) à distance configuré pour transmettre le signal numérique vers un terminal distant.

13. Dispositif électro-médical (10) selon la revendication 12, dans lequel, les moyens électroniques (50) comportent une mémoire interne (53) configurée pour stocker le signal numérique converti.

14. Dispositif électro-médical selon l'une des revendications 1 à 13, qui comporte, une interface homme/machine (56) ménagée sur une paroi (21, 22, 22a) du boitier (20), l'interface homme/machine (56) comprenant un ou plusieurs boutons (560) et/ou un ou plusieurs indicateurs lumineux (561, 562, 563).

15. Kit de mesure (90) de données physiologiques d'un patient telles que la pression intracrânienne ou la pression artérielle d'un patient, le kit de mesure (90) comprenant un dispositif électro-médical (10) défini selon l'une des revendications 1 à 14 et un capteur physiologique (13) configuré, d'une part, pour mesurer la pression d'un liquide physiologique d'un patient, et d'autre part, pour produire un signal électrique analogique représentatif de ladite pression.
